(19) **Europäisches Patentamt / European Patent Office / Office européen des brevets**

(11) **EP 4 494 637 A1**

(12) **EUROPEAN PATENT APPLICATION**
published in accordance with Art. 153(4) EPC

(43) Date of publication:
**22.01.2025 Bulletin 2025/04**

(21) Application number: **23770824.3**

(22) Date of filing: **15.03.2023**

(51) International Patent Classification (IPC):
$A61K\ 31/09^{(2006.01)}$   $A23L\ 5/00^{(2016.01)}$
$A23L\ 29/10^{(2016.01)}$   $A23L\ 33/10^{(2016.01)}$
$A61K\ 9/14^{(2006.01)}$   $A61K\ 47/14^{(2017.01)}$
$A61K\ 47/22^{(2006.01)}$   $A61K\ 47/24^{(2006.01)}$
$A61K\ 47/36^{(2006.01)}$   $A61K\ 47/38^{(2006.01)}$
$A61P\ 3/00^{(2006.01)}$   $A61P\ 9/00^{(2006.01)}$
$A61P\ 39/06^{(2006.01)}$   $A61P\ 43/00^{(2006.01)}$

(52) Cooperative Patent Classification (CPC):
**A23L 5/00; A23L 29/10; A23L 33/10; A61K 9/14;
A61K 31/09; A61K 47/14; A61K 47/22;
A61K 47/24; A61K 47/36; A61K 47/38; A61P 3/00;
A61P 9/00; A61P 39/06; A61P 43/00**

(86) International application number:
**PCT/JP2023/010017**

(87) International publication number:
**WO 2023/176871 (21.09.2023 Gazette 2023/38)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **17.03.2022  JP 2022042302**

(71) Applicant: **Kaneka Corporation
Osaka-shi, Osaka 530-8288 (JP)**

(72) Inventors:
• **FUKUYAMA Yuka
  Takasago-shi, Hyogo 676-8688 (JP)**
• **KITAMURA Shiro
  Osaka-shi, Osaka 530-8288 (JP)**
• **YOKOCHI Yuichi
  Takasago-shi, Hyogo 676-8688 (JP)**

(74) Representative: **Hoffmann Eitle
Patent- und Rechtsanwälte PartmbB
Arabellastraße 30
81925 München (DE)**

(54) **SOLID COMPOSITION CONTAINING FORMII-TYPE CRYSTAL OF REDUCED COENZYME Q10**

(57)    The present specification discloses a means capable of controlling the decrease of reduced coenzyme Q10 due to its oxidation during storage of a solid composition containing the reduced coenzyme Q10. One or more embodiments of the present invention relate to a solid composition containing a reduced coenzyme Q10 Form II crystal, an emulsifier, and an antioxidant. Another one or more embodiments of the present invention relate to a method for producing a particulate solid composition containing a reduced coenzyme Q10 Form II crystal, an emulsifier, an antioxidant, and a binder, the method including granulating a mixture containing the reduced coenzyme Q10 Form II crystal, the emulsifier, the antioxidant, the binder, and a liquid binder.

EP 4 494 637 A1

**Description**

Technical Field

One or more embodiments of the present invention relate to a solid composition containing a reduced coenzyme Q10 Form II crystal.

Another one or more embodiments of the present invention relate to a method for producing a particulate solid composition containing a reduced coenzyme Q10 Form II crystal.

Background Art

Coenzyme Q is an essential component widely distributed in living organisms from bacteria to mammals, and is known as a member of the mitochondrial electron transfer system in cells in living organisms. In humans, the major form of coenzyme Q is coenzyme Q10, the side chain of which has ten repeating structures. In the living body, approximately 40% to 90% of the coenzyme Q10 is generally present in reduced form. The physiological activity of coenzyme Q includes activation of energy production by mitochondrial activation, activation of cardiac function, stabilization of cell membranes, and protection of cells by antioxidant activity.

While coenzyme Q10 currently produced and sold is, in large part, oxidized coenzyme Q10, reduced coenzyme Q10 (hereinafter sometimes referred to as "QH") which exhibits higher oral absorbability than oxidized coenzyme Q10 has also been commercially available and has come to be used in recent years.

Reduced coenzyme Q10 is easily oxidized, and therefore has the problems of high storage costs and limited scope of application to commercial forms. In particular, there is a demand for a solid composition that contains reduced coenzyme Q10, has excellent oxidation stability, and can be applied to a form such as a granule, hard capsule, or tablet.

Patent Literature 1 discloses that a solid composition containing reduced coenzyme Q10 is coated with a coating material such as shellac, gelatin, or gum arabic to be thereby stabilized.

Patent Literature 2 discloses a particulate composition in which an oily component containing reduced coenzyme Q10 is polydispersed in domain form in a matrix containing a water-soluble excipient and a water-soluble ascorbic acid.

Patent Literature 3 discloses that crystal polymorphism is found in reduced coenzyme Q10, and that a newly appearing crystalline form (this crystal is hereinafter referred to as "reduced coenzyme Q10 Form II crystal", "QH Form II crystal", or "Form II crystal") is much more stable than the conventional reduced coenzyme Q10 (this crystal is hereinafter referred to as a "reduced coenzyme Q10 Form I crystal", "QH Form I crystal", or "Form I crystal"), and also has other excellent physical properties.

Patent Literature 4 describes a reduced coenzyme Q10 composition that is suited for an inexpensive simple formulation capable of meeting market demand, has good oxidation stability, and is in a solid state such as a powder at room temperature, wherein the composition contains reduced coenzyme Q10, an organic acid, and a carbonate containing a sodium cation and/or a calcium cation, and is solid at 25°C. Patent Literature 4 discloses that the reduced coenzyme Q10 is preferably a Form II crystal. Patent Literature 4 discloses that, to improve the oxidation stability of the reduced coenzyme Q10 in the composition, it is essential to incorporate a carbonate containing a sodium cation and/or a calcium cation.

Patent Literature 5 describes a novel granule that can adsorb and solidify a large amount of a hardly soluble functional substance, and allow the functional substance to have excellent elution ability in water, wherein the granule contains: a support containing a porous calcium silicate; a functional substance and a nonionic surfactant that are adsorbed onto the support; and a binder, and wherein the functional substance is at least one selected from the group consisting of: a hardly soluble liquid; a hardly soluble solid having a low melting point; and a crude-drug extract containing a hardly soluble active ingredient. In Patent Literature 5, coenzyme Q10 is described as a specific example of a functional substance.

Patent Literature 6 describes a method for producing a particulate product containing an emulsifier by tumbling granulation, but discloses that granulation using an emulsifier having a melting point lower than 25°C, i.e., an emulsifier that is liquid at ordinary temperature, causes the granulated product to form lumps, often failing to give a desired granulated product.

Citation List

Patent Literature

Patent Literature 1: WO 2006/075502

Patent Literature 2: WO 2008/129980
Patent Literature 3: WO 2012/176842
Patent Literature 4: WO 2015/122531
Patent Literature 5: JP 2010-189337 A
Patent Literature 6: JP S60-221078 A

Summary of Invention

Technical Problem

However, such a solid composition containing reduced coenzyme Q10 as conventionally disclosed is produced by a complicated production method, and there is still room for improvement in the oxidation stability of reduced coenzyme Q10.

Production of the solid composition in Patent Literature 1 requires a step of spraying a coating material solution and a subsequent step of drying. In addition, production of the particulate composition in Patent Literature 2 requires: a step of preparing an oil-in-water emulsified product with an aqueous solution of a water-soluble excipient such as gum arabic and with an oily component containing reduced coenzyme Q10; and a step of removing water from droplets of the emulsified composition by spray-drying or oil-heating to form the composition into particles.

When a coating material such as a water-soluble excipient is used to give gas barrier properties to a solid composition containing reduced coenzyme Q10, the solid composition needs to be completely coated, which leads to various problems such as limited production method, higher costs, larger size of the solid composition, or less amount of reduced coenzyme Q10 contained in the solid composition. Furthermore, the solid composition containing reduced coenzyme Q10 produced by the above-described method will crack under pressure, resulting in a loss of gas barrier properties, and is therefore not suitable as a raw material for further formulation such as tableting.

The reduced coenzyme Q10 Form II crystal in Patent Literature 3 exhibits a higher oxidation stability than a conventional Form I crystal, but is desired to be further stabilized in order to be formed into a hard capsule or a tablet. In addition, the compositions in Patent Literature 4 and 5 still have room for improvement in stabilizing reduced coenzyme Q10. Besides, the compositions require carbonate and porous calcium silicate as essential components, which limits their use in some cases.

The present specification discloses a means capable of controlling the decrease of reduced coenzyme Q10 due to its oxidation during storage of a solid composition containing the reduced coenzyme Q10.

Solution to Problem

The present inventors have completed each of the below-described embodiments of the present invention through the new discovery that, a solid composition containing a reduced coenzyme Q10 Form II crystal together with an emulsifier and an antioxidant, such as a solid composition obtained by granulating a reduced coenzyme Q10 Form II crystal and a solid antioxidant in the presence of a specific emulsifier using a granulation method with pressure or a fluidized-bed granulation method, allows to control the decrease of the reduced coenzyme Q10 in the solid composition during storage due to oxidation.

(1) A solid composition containing a reduced coenzyme Q10 Form II crystal, an emulsifier, and an antioxidant.
(2) The solid composition according to (1), wherein the emulsifier is one or more selected from the group consisting of: ester compounds of polyols and fatty acids optionally having substituents, wherein the polyols are selected from the group consisting of monoglycerol, polyglycerol, sorbitan, polyoxyethylene sorbitan, sucrose, propylene glycol, polypropylene glycol, ethylene glycol, and polyethylene glycol; and lecithin.
(3) The solid composition according to (1) or (2), containing 1 part by weight or more and 9900 parts by weight or less of the emulsifier with respect to 100 parts by weight of the crystal.
(4) The solid composition according to any one of (1) to (3), wherein the amount of the emulsifier contained in the solid composition is 1% by weight or more and 99% by weight or less.
(5) The solid composition according to any one of (1) to (4), wherein the antioxidant is one or more selected from the group consisting of ascorbic acid, ascorbic acid salts, erythorbic acid, and erythorbic acid salts.
(6) The solid composition according to any one of (1) to (5), further containing a binder.
(7) The solid composition according to (6), wherein the binder is one or more selected from the group consisting of celluloses and starches.
(8) The solid composition according to (7), wherein the binder is one or more selected from the group consisting of hydroxypropyl cellulose and hydroxypropylmethyl cellulose.
(9) The solid composition according to any one of (6) to (8), wherein the solid composition is a particulate solid composition.

(10) The solid composition according to any one of (1) to (9), wherein the solid composition is free of a carbonate containing a sodium cation and/or a calcium cation.

(11) The solid composition according to any one of (1) to (10), wherein the solid composition is free of porous calcium silicate.

(12) A method for producing a particulate solid composition containing a reduced coenzyme Q10 Form II crystal, an emulsifier, an antioxidant, and a binder,

the method including granulating a mixture containing the reduced coenzyme Q10 Form II crystal, the emulsifier, the antioxidant, the binder, and a liquid binder.

(13) The method according to (12), wherein the granulating is performed by an extrusion granulation method, a high-shear granulation method, or a fluidized-bed granulation method.

The present specification encompasses the disclosure of Japanese Patent Application No. 2022-042302 that serves as a basis for the priority of the present application.

Advantageous Effects of Invention

In the solid composition according to one or more embodiments of the present invention, the decrease of reduced coenzyme Q10 due to its oxidation during storage can be controlled.

The method according to another one or more embodiments of the present invention can produce a particulate solid composition containing a reduced coenzyme Q10 Form II crystal and having excellent storage stability.

In addition, the method according to one or more embodiments of the present invention can produce a particulate solid composition containing a reduced coenzyme Q10 Form II crystal and having excellent handling properties at a higher yield.

Description of Embodiments

Hereinafter, the present invention will be described in detail.

<Reduced Coenzyme Q10>

The reduced coenzyme Q10 herein may partially include oxidized coenzyme Q10 as long as the reduced coenzyme Q10 is included as a main component. The main component herein means that it is included in a percentage of, for example, 50% by weight or more, typically 60% by weight or more, preferably 70% by weight or more, more preferably 80% by weight or more, still more preferably 90% by weight or more, particularly preferably 95% by weight or more, and particularly 98% by weight or more. The above-described percentages are the percentages of the reduced coenzyme Q10 to the total weight of coenzyme Q10.

As described above, the reduced coenzyme Q10 encompasses two forms of crystal polymorphisms, i.e., Form I and Form II. Specifically, the crystalline form of reduced coenzyme Q10 having a melting point around 48°C and showing characteristic peaks at diffraction angles ($2\theta \pm 0.2°$) of 3.1°, 18.7°, 19.0°, 20.2°, and 23.0° in powder X-ray (Cu-K$\alpha$) diffraction is a Form I crystal, whereas the crystalline form of reduced coenzyme Q10 having a melting point around 52°C and showing characteristic peaks at diffraction angles ($2\theta \pm 0.2°$) of 11.5°, 18.2°, 19.3°, 22.3°, 23.0°, and 33.3° in powder X-ray (Cu-K$\alpha$) diffraction is a Form II crystal.

One or more embodiments of the present invention are characterized by using reduced coenzyme Q10 (QH) in the form of a reduced coenzyme Q10 Form II crystal (QH Form II crystal). The QH Form II crystal has high stability to oxidation, and thus, a solid composition containing the QH Form II crystal together with an emulsifier and an antioxidant has excellent storage stability.

The QH Form II crystal may be composed of a Form II crystal alone, or may be a QH crystal or crystalline solid containing a Form II crystal as a main component. Here, the main component means that the amount of the Form II crystal is preferably 80% by weight or more, more preferably 90% by weight or more, more preferably 95% by weight or more, most preferably 98% by weight or more, with respect to the total amount of QH.

<Emulsifier>

Herein, the emulsifier is not limited to any kind. For example, an emulsifier having an HLB of 1 or more and 17 or less, preferably 2 or more and 16 or less, may be used. Two or more kinds of emulsifiers may be used in combination.

Among the above-described emulsifiers, an emulsifier that is not in the form of powder or flakes is preferable from the viewpoint of the effect of controlling the oxidation of reduced coenzyme Q10. Examples of the emulsifier that is not in the form of powder or flakes include emulsifiers that are in the form of liquid, sol, gel, or soft solid. An emulsifier that is in the form of liquid, viscous liquid, sticky liquid, paste, pellets, waxy lump, wax, soft solid, or semisolid is more preferable. The emulsifier is preferably in the above-described form at 50°C, most preferably in the above-described form at 25°C.

Examples of the emulsifier that is not in the form of powder or flakes include an emulsifier having a melting starting point of 50°C or less, preferably 40°C or less, more preferably 25°C or less, as measured with a differential scanning calorimeter (DSC) set at a heating rate of 1°C/min or more and 20°C/min or less.

From another viewpoint, the emulsifier may be, for example, an emulsifier having a viscosity of 150,000 mPa·s or less, preferably 100,000 mPa·s or less, 50,000 mPa·s or less, more preferably 30,000 mPa·s or less, most preferably 25,000 mPa·s or less, as measured using a Brookfield type viscometer at a rotational rate of 10 rpm at a sample temperature of 50°C. The lower limit of the viscosity is not particularly limited, as long as it is greater than 0 mPa·s, and is more preferably 1 mPa·s or more, more preferably 5 mPa·s or more, most preferably 10 mPa·s or more.

In one or more embodiments of the present invention, a particulate solid composition obtained using the above-described emulsifier that is not in the form of powder or flakes retains good handling properties (dischargeability from a granulator, mesh passability, conveyability, and the like) contrary to the suggestion of Patent Literature 6 or the like, resulting in the particulate solid composition with both oxidative stability of the reduced coenzyme Q10 contained and good handling properties.

Specific examples of the emulsifier include one or more selected from the group consisting of: ester compounds of polyols and fatty acids optionally having substituents, wherein the polyols are selected from the group consisting of monoglycerol, polyglycerol, sorbitan, polyoxyethylene sorbitan, sucrose, propylene glycol, polypropylene glycol, ethylene glycol, and polyethylene glycol; and lecithin.

The number of glycerol units in the polyglycerol may be 2 or more, and is preferably 2 or more and 10 or less. Examples include diglycerol, triglycerol, tetraglycerol, pentaglycerol, hexaglycerol, and decaglycerol.

The number of oxyethylene units in the polyoxyethylene sorbitan may be 2 or more, preferably 10 or more and 30 or less, more preferably 15 or more and 25 or less.

The number of propylene glycol units in the polypropylene glycol may be 2 or more, and is preferably 2 or more and 10 or less.

The number of ethylene glycol units in the polyethylene glycol may be 2 or more, and is preferably 2 or more and 10 or less.

Examples of the fatty acid optionally having a substituent include linear or branched, monovalent or divalent fatty acids having 4 or more and 24 or less carbon atoms.

Examples of the substituent include a hydroxyl group and an acetoxy group. The number of the substituents is preferably 2 or less. Specific examples of the fatty acid optionally having a substituent include lauric acid, oleic acid, caprylic acid, stearic acid, behenic acid, ricinoleic acid, succinic acid, and diacetyl tartaric acid.

In the ester compound of the polyol and the fatty acid, the number of fatty acids bound to one polyol molecule is not particularly limited, and may be appropriately adjusted in accordance with the intended HLB of the emulsifier.

From the viewpoint of controlling the oxidation of reduced coenzyme Q10, the number of fatty acids bound to one polyol molecule may be, for example, 12 or less, preferably 10 or less, more preferably 7 or less, more preferably 6 or less, more preferably 5 or less, more preferably 3 or less, more preferably 2 or less, more preferably 1.

Specific examples of the ester compound of the polyol and the fatty acid include diglycerol monooleate, monoglycerol monocaprylate, diglycerol monocaprylate, decaglycerol pentaoleate, tetraglycerol pentaoleate, pentaglycerol trioleate, decaglycerol monolaurate, hexaglycerol monocaprylate, hexaglycerol monooleate, pentaglycerol monostearate, tetraglycerol tristearate, decaglycerol monobehenate, mono- and di-glycerol monostearates, monoglycerol monooleate, glycerol monostearate succinate, monoglycerol succinate, glycerol monostearate diacetyl tartrate, propylene glycol monooleate, sorbitan monooleate, sorbitan monostearate, sorbitan tristearate, monoglycerol monolaurate, diglycerol monolaurate, diglycerol monomyristate, tetraglycerol pentastearate, polyoxyethylene sorbitan monooleate, polyoxyethylene sorbitan monolaurate, polyoxyethylene sorbitan monostearate, condensed pentaglycerol ricinoleate, sucrose stearate, sucrose erucate, and sucrose oleate.

In the ester compound of the polyol and the fatty acid, the fatty acid is more preferably an unsaturated fatty acid from the viewpoint of controlling the oxidation of reduced coenzyme Q10. Examples of the unsaturated fatty acid include: crotonic acid, myristoyleic acid, palmitoleic acid, sapienic acid, oleic acid, elaidic acid, vaccenic acid, gadoleic acid, eicosenoic acid, erucic acid, nervonic acid, linoleic acid, eicosadienoic acid, docosadienoic acid, α-linolenic acid, γ-linolenic acid, pinolenic acid, α-eleostearic acid, β-eleostearic acid, mead acid, dihomo-γ-linolenic acid, eicosatrienoic acid, stearidonic acid, arachidonic acid, eicosatetraenoic acid, adrenic acid, bosseopentaenoic acid, eicosapentaenoic acid, osbond acid, clupanodonic acid, tetracosapentaenoic acid, docosahexaenoic acid, nisinic acid, ricinoleic acid, and condensed ricinoleic acid; more preferably oleic acid, condensed ricinoleic acid, linoleic acid, and erucic acid; particularly preferably oleic acid.

From another viewpoint of controlling the oxidation of reduced coenzyme Q10, the ester compound of the polyol and the fatty acid is more preferably a glycerol fatty acid ester or a polyglycerol fatty acid ester that satisfies any of the following:

1) a glycerol fatty acid ester or a polyglycerol fatty acid ester having 1 or more fatty acid residues in which the fatty acid has 8 or less carbon atoms;

2) a polyglycerol fatty acid ester with 2 or more glycerol units having 1 or more fatty acid residues in which the fatty acid has 9 or more and 14 or less carbon atoms; and

3) a polyglycerol fatty acid ester with 5 or more glycerol units having 1 or more fatty acid residues in which the fatty acid has 15 or more and 18 or less carbon atoms.

In these cases, the fatty acid may be a saturated fatty acid or an unsaturated fatty acid.

Specific examples of the ester compound of the polyol and the unsaturated fatty acid include monoglycerol monooleate, monoglycerol mono- and di-oleates, monoglycerol dioleate, mono- and di-glycerol monooleates, diglycerol monooleate, diglycerol mono- and di-oleates, diglycerol dioleate, diglycerol trioleate, triglycerol monooleate, triglycerol dioleate, triglycerol trioleate, triglycerol tetraoleate, tetraglycerol monooleate, tetraglycerol dioleate, tetraglycerol trioleate, tetraglycerol tetraoleate, tetraglycerol pentaoleate, pentaglycerol monooleate, pentaglycerol dioleate, pentaglycerol trioleate, pentaglycerol tetraoleate, pentaglycerol pentaoleate, pentaglycerol hexaoleate, hexaglycerol monooleate, hexaglycerol dioleate, hexaglycerol trioleate, hexaglycerol tetraoleate, hexaglycerol pentaoleate, hexaglycerol hexaoleate, hexaglycerol heptaoleate, decaglycerol monooleate, decaglycerol dioleate, decaglycerol trioleate, decaglycerol tetraoleate, decaglycerol pentaoleate, decaglycerol hexaoleate, decaglycerol heptaoleate, decaglycerol octaoleate, decaglycerol nonaoleate, decaglycerol decaoleate, decaglycerol dodecaoleate, diacetoglycerol monooleate, glycerol monooleate lactate, glycerol monooleate succinate, glycerol monooleate citrate, glycerol monooleate diacetyl tartrate, sucrose oleate, propylene glycol monooleate, sorbitan monooleate, sorbitan dioleate, sorbitan trioleate, polyoxyethylene sorbitan monooleate, 1-palmitoyl-2-oleoylphosphatidylcholine, phosphatidylcholine dilinoleate, monoglycerol monoerucate, monoglycerol mono- and di-erucates, monoglycerol dierucate, mono- and di-glycerol monoerucates, diglycerol monoerucate, diglycerol mono- and di-dierucates, diglycerol dierucate, diglycerol trierucate, triglycerol monoerucate, triglycerol dierucate, triglycerol trierucate, triglycerol tetraerucate, tetraglycerol monoerucate, tetraglycerol dierucate, tetraglycerol trierucate, tetraglycerol tetraerucate, tetraglycerol pentaerucate, pentaglycerol monoerucate, pentaglycerol dierucate, pentaglycerol trierucate, pentaglycerol tetraerucate, pentaglycerol pentaerucate, pentaglycerol hexaerucate, hexaglycerol monoerucate, hexaglycerol dierucate, hexaglycerol trierucate, hexaglycerol tetraerucate, hexaglycerol pentaerucate, hexaglycerol hexaerucate, hexaglycerol heptaerucate, decaglycerol monoerucate, decaglycerol dierucate, decaglycerol trierucate, decaglycerol tetraerucate, decaglycerol pentaerucate, decaglycerol hexaerucate, decaglycerol heptaerucate, decaglycerol octaerucate, decaglycerol nonaerucate, decaglycerol decaerucate, decaglycerol dodecaerucate, diacetoglycerol monoerucate, glycerol monoerucate lactate, glycerol monoerucate succinate, glycerol monoerucate citrate, glycerol monoerucate diacetyl tartrate, sucrose erucate, propylene glycol monoerucate, sorbitan monoerucate, sorbitan dierucate, sorbitan trierucate, condensed monoglycerol ricinoleate, condensed diglycerol ricinoleate, condensed triglycerol ricinoleate, condensed tetraglycerol ricinoleate, condensed pentaglycerol ricinoleate, condensed hexaglycerol ricinoleate, condensed heptaglycerol ricinoleate, and condensed decaglycerol ricinoleate.

Specific examples of the ester compound of the polyol and the unsaturated fatty acid include: preferably monoglycerol monooleate, monoglycerol mono- and di-oleates, monoglycerol dioleate, mono- and di-glycerol monooleates, diglycerol monooleate, diglycerol mono- and di-oleates, diglycerol dioleate, diglycerol trioleate, triglycerol monooleate, triglycerol dioleate, triglycerol trioleate, triglycerol tetraoleate, tetraglycerol monooleate, tetraglycerol dioleate, tetraglycerol trioleate, tetraglycerol tetraoleate, tetraglycerol pentaoleate, pentaglycerol monooleate, pentaglycerol dioleate, pentaglycerol trioleate, pentaglycerol tetraoleate, pentaglycerol pentaoleate, pentaglycerol hexaoleate, hexaglycerol monooleate, hexaglycerol dioleate, hexaglycerol trioleate, hexaglycerol tetraoleate, hexaglycerol pentaoleate, hexaglycerol hexaoleate, hexaglycerol heptaoleate, decaglycerol monooleate, decaglycerol dioleate, decaglycerol trioleate, decaglycerol tetraoleate, decaglycerol pentaoleate, decaglycerol hexaoleate, decaglycerol heptaoleate, decaglycerol octaoleate, decaglycerol nonaoleate, decaglycerol decaoleate, decaglycerol dodecaoleate, diacetoglycerol monooleate, glycerol monooleate lactate, glycerol monooleate succinate, glycerol monooleate citrate, glycerol monooleate diacetyl tartrate, sucrose oleate, propylene glycol monooleate, sorbitan monooleate, sorbitan dioleate, sorbitan trioleate, polyoxyethylene sorbitan monooleate, 1-palmitoyl-2-oleoylphosphatidylcholine; particularly preferably diglycerol monooleate.

From the viewpoint of controlling the oxidation of reduced coenzyme Q10, a polyoxyethylene sorbitan fatty acid ester is also preferable as the ester compound of the polyol and the fatty acid.

Specific examples of the polyoxyethylene sorbitan fatty acid ester include polyoxyethylene sorbitan monooleate, polyoxyethylene sorbitan monolaurate, and polyoxyethylene sorbitan monostearate.

When the solid composition containing the emulsifier is used in combination with an oily component (for example, vegetable oil, essential oil, animal oil or fat, fish oil, a lipid-soluble active component, or the like), the emulsifier contained in the solid composition preferably has an HLB of less than 10.0, more preferably an HLB of less than 8.0, particular preferably an HLB of less than 6.0. The use of the ester composed of the polyol and the unsaturated fatty acid and having an HLB of less than 6.0 as the emulsifier allows the reduced coenzyme Q10 in the solid composition to be stably stored even under conditions of a relative humidity of less than 50%, i.e., conditions under which pharmaceutical products and foods are often stored, and is therefore preferable.

Specific examples of the ester composed of the polyol and the unsaturated fatty acid and having an HLB of less than 6.0 include monoglycerol monooleate, monoglycerol mono- and di-oleates, monoglycerol dioleate, mono- and di-glycerol monooleates, tetraglycerol pentaoleate, hexaglycerol pentaoleate, decaglycerol pentaoleate, decaglycerol decaoleate, sorbitan monooleate, sorbitan trioleate, propylene glycol monooleate, monoglycerol monooleate citrate, sucrose oleate, decaglycerol erucate, and sucrose erucate.

Examples of the lecithin include soybean lecithin, egg yolk lecithin, and enzymolytic soybean lecithin.

The emulsifier to be used is particularly preferably an emulsifier acceptable for foods, cosmetics, and/or pharmaceutical products.

<Antioxidant>

Herein, the antioxidant is not limited to any kind. Two or more kinds of antioxidants may be used in combination. The antioxidant is preferably solid at ordinary temperature, and specific examples include one or more selected from the group consisting of ascorbic acid, ascorbic acid salts, erythorbic acid, and erythorbic acid salts.

The counterion of each of the ascorbic acid salt and the erythorbic acid salt is not limited, and the ascorbic acid salt and the erythorbic acid salt may be independently one or more metal salts selected from the group consisting of sodium salts, potassium salts, calcium salts, and magnesium salts.

The antioxidant to be used is particularly preferably an antioxidant acceptable for foods or pharmaceutical products. The antioxidant is preferably an ascorbic acid salt, particularly preferably one or more selected from the group consisting of sodium ascorbate and calcium ascorbate.

<Binder>

The solid composition disclosed herein preferably further contains a binder. The binder can be used for binding components including the reduced coenzyme Q10 Form II crystal, the emulsifier, and the antioxidant to form a particulate solid composition.

The binder is not limited to any kind. Two or more kinds of binders may be used in combination. Specific examples of the binder include one or more selected from the group consisting of celluloses and starches.

Examples of the celluloses include hydroxypropyl cellulose, hydroxypropylmethyl cellulose, hydroxymethyl cellulose, carboxymethyl cellulose, crystalline cellulose, powdered cellulose, methyl cellulose, ethyl cellulose, and salts thereof. The binder is particularly preferably one or more selected from the group consisting of hydroxypropyl cellulose, hydroxypropylmethyl cellulose, and sodium carboxymethyl cellulose.

Examples of the starches include wheat starch, potato starch, sweet potato starch, corn starch, dextrin, hydroxypropyl starch, starch acetate, oxidized starch, octenyl succinate starch and salts thereof, and partially pregelatinized starch.

The binder to be used is particularly preferably a binder acceptable for foods or pharmaceutical products.

<Solid Composition>

One or more embodiments of the present invention relate to a solid composition containing the reduced coenzyme Q10 Form II crystal, the emulsifier, and the antioxidant.

Even when stored under conditions involving contact with air, the solid composition according to the present embodiment allows to control the decrease of the reduced coenzyme Q10 due to oxidation and is thus suitable for storage for a long time, for example, a period of three months or more. The storage temperature for the solid composition is, for example, 0°C or more and 45°C or less, preferably 0°C or more and 40°C or less, more preferably 5°C or more and 25°C or less. Under the condition of a temperature of 25°C or less, the solid composition according to the present embodiment can maintain the reduced coenzyme Q10 in an amount of 90% by weight or more of the initial amount for a period as long as 20 months or more. In order to provide the solid composition in the above-described form at a reasonable price, it is preferable that the reduced coenzyme Q10 can be maintained in an amount of 90% by weight or more, more preferably 95% by weight or more, most preferably 97% by weight or more, of the initial amount for three months under conditions of 25°C together with silica gel in a hermetically sealed system, or for one month under conditions of 40°C together with silica gel in a hermetically sealed system.

The solid composition according to the present embodiment is a composition that is solid at 25°C. The solid composition according to the present embodiment may have any shape, such as a particulate, powdery, or flaky shape, and preferably has a particulate shape. The particulate shape may be any shape formed by binding primary particles of a material containing the reduced coenzyme Q10 Form II crystal, the emulsifier, and the antioxidant by granulation, and encompasses a granular shape. The particulate solid composition is not limited to any dimensions, and can be, for example, granules having a longest diameter of 0.20 mm or more and 2.0 mm or less.

The solid composition according to the present embodiment can be produced at a high yield and has good handling properties even when the emulsifier used is a liquid, sol, gel, or soft solid. For example, the solid composition produced by high-shear granulation exhibits a pass rate of 50% or more, preferably 60% or more, more preferably 70% or more, more preferably 80% or more, particularly preferably 90% or more, with respect to a mesh having an opening of 710 μm. In this regard, the size of the mesh opening is generally adjusted in accordance with an assumed particle diameter. For granules obtained by extrusion granulation, a mesh having an opening of 3.0 mm or the like can be used.

In addition, the solid composition according to the present embodiment contains reduced coenzyme Q10 in a crystalline form. The crystallinity of reduced coenzyme Q10 (the ratio of the amount of the reduced coenzyme Q10 in the crystalline form to the total amount of the reduced coenzyme Q10 contained in the solid composition) is typically 50% or more, preferably 70% or more, more preferably 90% or more, particularly preferably 95% or more.

The crystallinity of the reduced coenzyme Q10 contained in the solid composition can be determined by measuring the heat of melting of the crystal by DSC analysis, then calculating the crystallinity in accordance with the formula described below using the measured heat of melting and the theoretical heat of melting determined from the amount of the reduced coenzyme Q10 contained in the solid composition.

$$\text{Crystallinity (\%)} = (\text{Measured heat of melting/Theoretical heat of melting}) \times 100$$

Additionally, in the solid composition according to the present embodiment, the reduced coenzyme Q10 does not need to be completely coated with a gas-barrier material. Accordingly, the solid composition does not have its oxidation stability compromised by coating breakage even under pressure and can therefore be applied to a wide variety of formulation forms, such as compression tablets.

In the solid composition according to the present embodiment, the ratio of each component can be adjusted so that the oxidation of the reduced coenzyme Q10 can be controlled.

The solid composition according to the present embodiment more preferably contains the antioxidant, for example, at 1 part by weight or more and 9900 parts by weight or less with respect to 100 parts by weight of the reduced coenzyme Q10 Form II crystal. The lower limit of the amount of the antioxidant with respect to 100 parts by weight of the reduced coenzyme Q10 Form II crystal is preferably 20 parts by weight or more, more preferably 50 parts by weight or more, more preferably 80 parts by weight or more. The upper limit of the amount of the antioxidant with respect to 100 parts by weight of the reduced coenzyme Q10 Form II crystal is preferably 5000 parts by weight or less, more preferably 1000 parts by weight or less, more preferably 500 parts by weight or less, more preferably 200 parts by weight or less, more preferably 120 parts by weight or less.

The solid composition according to the present embodiment more preferably contains the emulsifier, for example, at 1 part by weight or more and 9900 parts by weight or less with respect to 100 parts by weight of the reduced coenzyme Q10 Form II crystal. The lower limit of the amount of the emulsifier with respect to 100 parts by weight of the reduced coenzyme Q10 Form II crystal is preferably 3 parts by weight or more, more preferably 5 parts by weight or more, more preferably 8 parts by weight or more. The upper limit of the amount of the emulsifier with respect to 100 parts by weight of the reduced coenzyme Q 10 Form II crystal is preferably 5000 parts by weight or less, more preferably 1000 parts by weight or less, more preferably 500 parts by weight or less, more preferably 200 parts by weight or less, more preferably 150 parts by weight or less, more preferably 100 parts by weight or less, more preferably 50 parts by weight or less, more preferably 30 parts by weight or less.

The lower limit of the amount of the reduced coenzyme Q10 Form II crystal contained in the solid composition according to the present embodiment is, for example, 1% by weight or more, preferably 10% by weight or more, more preferably 20% by weight or more, more preferably 30% by weight or more. The upper limit of the amount of the reduced coenzyme Q10 Form II crystal is, for example, 90% by weight or less, preferably 80% by weight or less, more preferably 70% by weight or less, more preferably 60% by weight or less. In the solid composition according to the present embodiment, the reduced coenzyme Q10 does not need to be completely coated with a gas-barrier material. Thus, a larger amount of the reduced coenzyme Q10 can be contained, and the amount of the reduced coenzyme Q10 Form II crystal can be, for example, 45% or more, 50% or more, 55% or more, 60% or more, 70% or more, or 80% or more.

The lower limit of the amount of the antioxidant contained in the solid composition according to the present embodiment is, for example, 1% by weight or more, preferably 10% by weight or more, more preferably 20% by weight or more, more preferably 30% by weight or more. The upper limit of the amount of the antioxidant is, for example, 99% by weight or less, preferably 90% by weight or less, preferably 80% by weight or less, more preferably 70% by weight or less, more preferably 60% by weight or less.

The amount of the emulsifier contained in the solid composition according to the present embodiment is not particularly limited. The lower limit of the amount of the emulsifier is, for example, 0.5% by weight or more, preferably 1% by weight or more, more preferably 3% by weight or more. The upper limit of the amount of the emulsifier is, for example, 99% by weight or less, preferably 50% by weight or less, more preferably 30% by weight or less, more preferably 25% by weight or less, more preferably 20% by weight or less, more preferably 15% by weight or less, more preferably 10% by weight or less. When the emulsifier used is in a form other than powder or flakes, for example, one or more forms selected from the group consisting of liquid, sol, gel, and soft solid, it is conventionally difficult to obtain the solid composition having good handling properties at a high yield. However, when the amount of the emulsifier contained in the solid composition according to the present embodiment is, for example, 50% by weight or less, preferably 30% by weight or less, more preferably 25% by weight or less, it is possible to obtain the solid composition having good handling properties at a high yield.

When the solid composition according to the present embodiment contains the binder, the lower limit of the amount of the binder contained in the solid composition is, for example, 1% by weight or more, preferably 5% by weight or more, more preferably 8% by weight or more. The upper limit of the amount of the binder is, for example, 20% by weight or less, preferably 15% by weight or less, more preferably 10% by weight.

The solid composition according to the present embodiment more preferably contains the binder, for example, at an amount of 1 part by weight or more, preferably 3 parts by weight or more, more preferably 5 parts by weight or more, more preferably 8 parts by weight or more, with respect to 100 parts by weight of the reduced coenzyme Q10 Form II crystal. In addition, the solid composition contains the antioxidant, for example, at an amount of 200 parts by weight or less, preferably 150 parts by weight or less, more preferably 100 parts by weight or less, more preferably 50 parts by weight or less, still more preferably 30 parts by weight or less, with respect to 100 parts by weight of the reduced coenzyme Q10 Form II crystal.

The solid composition according to the present embodiment may further contain another component such as an excipient. Another component is preferably a component acceptable for foods or pharmaceutical products.

The solid composition according to the present embodiment is preferably free of a carbonate containing a sodium cation and/or a calcium cation. Examples of the carbonate containing a sodium cation include sodium carbonate and hydrates thereof (for example, monohydrates, heptahydrates, decahydrates, and the like) and sodium bicarbonate. Examples of the carbonate containing a calcium cation include calcium carbonate and hydrates thereof (for example, 0.65-hydrates, monohydrates, 1.5-hydrates, hexahydrates, and the like).

The solid composition according to the present embodiment is preferably free of a porous calcium silicate. Examples of the porous calcium silicate include a calcium silicate having an average particle diameter of 18 to 32 μm, a loose bulk density of 0.07 to 0.15 g/mL, and an oil absorption rate of 300 to 550 mL/100 g.

<Method for Producing Particulate Solid Composition>

Another one or more embodiments of the present invention relate to a method for producing a particulate solid composition containing a reduced coenzyme Q10 Form II crystal, an emulsifier, an antioxidant, and a binder, the method including granulating a mixture containing the reduced coenzyme Q10 Form II crystal, the emulsifier, the antioxidant, the binder, and a liquid binder.

In the method according to the present embodiment, the reduced coenzyme Q10 crystal (Form II crystal) and a solid antioxidant are granulated in the presence of the emulsifier (which is preferably a liquid, sol, gel, or soft solid emulsifier), thereby producing the particulate solid composition characterized by having a high storage stability in a low cost and simple manner.

A granulation method can be appropriately selected. For example, an extrusion granulation method, high-shear granulation method, tumbling granulation method (rotation granulation method), dry granulation method, compression granulation method, powder joining method (particle composing, surface treatment and spheronization of dry powder by high shear), fluidized-bed granulation method, coacervation method, spray-dry method, cold-spray method, evaporation method, or in situ gelation coating method (orifice method) can be selected. The granulation method to be performed is preferably a granulation method with pressure (for example, extrusion granulation method, high-shear granulation method, tumbling granulation method, dry granulation method, compression granulation method, or powder joining method), or a fluidized-bed granulation method, more preferably an extrusion granulation method, high-shear granulation method, or fluidized-bed granulation method, particularly preferably an extrusion granulation method or high-shear granulation method.

In the method according to the present embodiment, the reduced coenzyme Q10 crystal is not completely dissolved or melted during a granulation process, and typically maintains its crystalline state during mixing and granulation.

The components other than a liquid binder in the mixture to be subjected to granulation can be a powdery mixture. The properties of each of the components other than the liquid binder in the mixture are as described for each of the components contained in a solid composition according to one or more embodiments of the present invention.

Examples of the liquid binder include water and ethanol. In addition, the liquid binder may be prepared by dissolving the above-described binder in a liquid such as water or ethanol. The liquid binder is particularly preferably water.

The granulation may be carried out under appropriate conditions to produce a particulate solid composition having a size suitable for the intended use.

The method according to the present embodiment preferably further includes a drying step of drying the granulated particulate solid composition to remove volatile components derived from the raw material components and the liquid binder. Additionally, if desired, a particulate solid composition having a desired particle diameter can be separated and collected by sieving or the like.

Examples

One or more embodiments of the present invention will be further specifically described with reference to the following Examples, but the present invention is not limited to these Examples. It should be noted that the HPLC measurement conditions in Examples and Comparative Examples are as described below.

(Method for Evaluating Oxidation Stability)

The weight ratio of the reduced coenzyme Q10 to the total coenzyme Q10 (i.e., reduced coenzyme Q10/(oxidized coenzyme Q10 + reduced coenzyme Q10)) is defined as a "QH ratio". The QH ratio was determined by the following HPLC analysis. For the evaluation of oxidation stability, a rate of the change in the QH ratio at the end of the evaluation from that at the beginning of the evaluation was defined as "QH residual rate", and the QH residual rate determined by the following formula was used as a measure of oxidation stability.

QH residual rate (%) = 100 × QH ratio at the end of the evaluation/QH ratio at the beginning of the evaluation

(HPLC Measurement Conditions)

Column: SYMMETRY $C_{18}$ (manufactured by Waters Corporation); 250 mm (in length) and 4.6 mm (in inner diameter)
Mobile phase: $C_2H_5OH:CH_3OH$ = 4:3 (v:v)
Detection wavelength: 210 nm
Flow rate: 1 mL/min

(Comparative Example 1)

A reduced coenzyme Q10 Form I crystal in an amount of 1.0 g, 1.0 g of sodium ascorbate, 0.2 g of hydroxypropyl cellulose, and 0.2 g of diglycerol monooleate (SUNSOFT Q-17D, manufactured by Taiyo Kagaku Co., Ltd.; having an HLB value of 7.5) were mixed, and 0.09 mL of water was added. The resulting mixture was kneaded. The kneaded material obtained was extrusion-granulated using a 1.2 mm Φ screen, and dried to obtain a particulate solid composition (granulated product). The resulting particulate solid composition in an amount of 1 g was stored at 25°C and 60%RH for two months, and the storage stability of the reduced coenzyme Q10 was evaluated on the basis of the QH residual rate. The results were as shown in Table 1.

[Table 1]

|  | QH Residual Rate (%) | | |
| --- | --- | --- | --- |
|  | 0.5 months | 1 month | 2 months |
| Comparative Example 1 | 65.1 | 52.0 | 48.4 |

As shown in Table 1, the reduced coenzyme Q10 in the composition containing the reduced coenzyme Q10 Form I crystal had a low storage stability.

(Comparative Example 2)

A reduced coenzyme Q10 Form II crystal in an amount of 1.0 g, 1.0 g of sodium ascorbate, and 0.2 g of hydroxypropyl cellulose were mixed, and 0.09 mL of water was added. The resulting mixture was kneaded. The kneaded material obtained was extrusion-granulated using a 1.2 mm Φ screen, and dried to obtain a particulate solid composition. The resulting particulate solid composition in an amount of 1 g was stored at 40°C and 75%RH for 6 months or at 25°C and 60%RH for 20 months, and the storage stability of the reduced coenzyme Q10 was evaluated on the basis of the QH residual rate.

(Example 1)

A reduced coenzyme Q10 Form II crystal in an amount of 1.0 g, 1.0 g of sodium ascorbate, 0.2 g of hydroxypropyl cellulose, and 0.2 g of any of the emulsifiers (A-1 to A-13) listed in Table 2 were mixed, and water was added. The resulting mixture was kneaded. The kneaded material obtained was extrusion-granulated using a 1.2 mm Φ screen, and dried to obtain a particulate solid composition. The resulting particulate solid composition in an amount of 1 g was stored at 25°C and 60%RH for 20 months or at 40°C and 75%RH for 2 to 6 months, and the storage stability of the reduced coenzyme Q10 was evaluated on the basis of the QH residual rate.

[Table 2]

| No | Name | | Product Name | HLB |
|---|---|---|---|---|
| A-1 | polyglycerol fatty acid ester | diglycerol monooleate | SUNSOFT Q-17D (manufactured by Taiyo Kagaku Co., Ltd.) | 7.5 |
| A-2 | | diglycerol monocaprylate | SUNSOFT Q-10D (manufactured by Taiyo Kagaku Co., Ltd.) | 9.5 |
| A-3 | | decaglycerol pentaoleate | SUNSOFT Q-175S (manufactured by Taiyo Kagaku Co., Ltd.) | 4.5 |
| A-4 | | pentaglycerol trioleate | SUNSOFT A-173E (manufactured by Taiyo Kagaku Co., Ltd.) | 7 |
| A-5 | | decaglycerol monolaurate | SUNSOFT M-12J (manufactured by Taiyo Kagaku Co., Ltd.) | 15.5 |
| A-6 | | hexaglycerol monocaprylate | SUNSOFT Q-81F (manufactured by Taiyo Kagaku Co., Ltd.) | 13 |
| A-7 | | hexaglycerol monooleate | SY-Glyster MO-5S (manufactured by Sakamoto Yakuhin Kogyo Co., Ltd.) | 11.6 |
| A-8 | distilled diglycerol fatty acid ester | diglycerol monooleate | DO-100V (manufactured by Riken Vitamin Co., Ltd.) | 7.4 |
| A-9 | higher fatty acid monoglyceride | monoglycerol monooleate | SUNSOFT No.8070V (manufactured by Taiyo Kagaku Co., Ltd.) | 4 |
| A-10 | sorbitan fatty acid ester | sorbitan monooleate | SUNSOFT No.81S (manufactured by Taiyo Kagaku Co., Ltd.) | 5.1 |
| A-11 | polyoxyethylene sorbitan fatty acid ester | polyoxyethylene sorbitan monooleate | Tween 80 (manufactured by DKS Co., Ltd) | 15 |
| A-12 | sucrose stearate | monoester: 20%, di-, tri-, and polyesters: 80% | Ryoto Sugar Ester S-370 (manufactured by Mitsubishi Chemical Corporation) | 3 |
| A-13 | lecithin | enzymolytic soybean lecithin | SUNLECITHIN A (manufactured by Taiyo Kagaku Co., Ltd.) | 12 |

The results of Comparative Example 2 and Example 1 are shown in Tables 3 and 4. Table 3 shows the QH residual rates after storage at 25°C for 20 months, and Table 4 shows the QH residual rates after storage at 40°C for 2 to 6 months.

Table 3

| | QH Residual Rate (%) |
|---|---|
| Comparative Example 2 | 92.6 |

(continued)

|  | QH Residual Rate (%) |
| --- | --- |
| Example 1 (A-1) | 96.0 |
| Example 1 (A-3) | 95.7 |
| Example 1 (A-11) | 93.1 |
| Example 1 (A-13) | 93.7 |

[Table 4]

|  | QH Residual Rate (%) | | |
| --- | --- | --- | --- |
|  | 2 months | 3 months | 6 months |
| Comparative Example 2 | 94.5 | 91.1 | 81.6 |
| Example 1 (A-2) | - | - | 92.1 |
|  | 95.7 | 94.9 | - |
| Example 1 (A-4) | 95.5 | 92.9 | - |
| Example 1 (A-5) | 97.6 | 96.7 | - |
| Example 1 (A-6) | - | - | 93.2 |
|  | 96.9 | 95.9 | - |
| Example 1 (A-7) | 96.9 | 96.4 | - |
| Example 1 (A-8) | 98.0 | 96.6 | - |
| Example 1 (A-9) | 96.5 | 95.4 | - |
| Example 1 (A-10) | 95.8 | 93.1 | - |
| Example 1 (A-12) | 95.2 | 92.0 | - |

As shown in Table 3 and Table 4, the reduced coenzyme Q10 in the compositions of Example 1, containing the antioxidant and the emulsifier together with the reduced coenzyme Q10 Form II crystal, had excellent storage stability.

(Example 2)

A reduced coenzyme Q10 Form II crystal in an amount of 1.0 g, 1.0 g of sodium erythorbate, 0.2 g of hydroxypropyl cellulose, and 0.2 g of diglycerol monooleate (SUNSOFT Q-17D, having an HLB value of 7.5) were mixed, and water was added. The resulting mixture was kneaded. The kneaded material obtained was extrusion-granulated using a 1.2 mm Φ screen, and dried to obtain a particulate solid composition. The resulting particulate solid composition in an amount of 1 g was stored at 40°C and 75%RH for three months, and the storage stability of the reduced coenzyme Q10 was evaluated on the basis of the QH residual rate.

(Example 3)

A reduced coenzyme Q10 Form II crystal in an amount of 1.0 g, 1.0 g of calcium ascorbate, 0.2 g of hydroxypropyl cellulose, and 0.2 g of diglycerol monooleate (SUNSOFT Q-17D, having an HLB value of 7.5) were mixed, and water was added. The resulting mixture was kneaded. The kneaded material obtained was extrusion-granulated using a 1.2 mm Φ screen, and dried to obtain a particulate solid composition. The resulting particulate solid composition in an amount of 1 g was stored at 40°C and 75%RH for three months, and the storage stability of the reduced coenzyme Q10 was evaluated on the basis of the QH residual rate.

(Example 4)

A reduced coenzyme Q10 Form II crystal in an amount of 1.0 g, 1.0 g of ascorbic acid, 0.2 g of hydroxypropyl cellulose, and 0.2 g of diglycerol monooleate (SUNSOFT Q-17D, having an HLB value of 7.5) were mixed, and water was added. The resulting mixture was kneaded. The kneaded material obtained was extrusion-granulated using a 1.2 mm Φ screen, and dried to obtain a particulate solid composition. The resulting particulate solid composition in an amount of 1 g was stored at 40°C and 75%RH for three months, and the storage stability of the reduced coenzyme Q10 was evaluated on the basis of the QH residual rate.

The QH residual rates after storage at 40°C for three months in Example 1 (in which the emulsifier A-1 was used), Example 2, Example 3, Example 4, and Comparative Example 2 are shown in Table 5.

[Table 5]

|  | Emulsifier | Antioxidant | QH Residual Rate (%) |
|---|---|---|---|
| Comparative Example 2 | none | sodium ascorbate | 91.1 |
| Example 1 (A-1) | diglycerol monooleate | sodium ascorbate | 95.3 |
| Example 2 | diglycerol monooleate | sodium erythorbate | 96.1 |
| Example 3 | diglycerol monooleate | calcium ascorbate | 97.2 |
| Example 4 | diglycerol monooleate | ascorbic acid | 96.0 |

(Example 5)

A reduced coenzyme Q10 Form II crystal in an amount of 1.0 g, 1.0 g of sodium ascorbate, 0.2 g of hydroxypropylmethyl cellulose, and 0.2 g of diglycerol monooleate (SUNSOFT Q-17D, having an HLB value of 7.5) were mixed, and water was added. The resulting mixture was kneaded. The kneaded material obtained was extrusion-granulated using a 1.2 mm Φ screen, and dried to obtain a particulate solid composition. The resulting particulate solid composition in an amount of 1 g was stored at 40°C and 75%RH for two months, and the storage stability of the reduced coenzyme Q10 was evaluated on the basis of the QH residual rate.

(Example 6)

A reduced coenzyme Q10 Form II crystal in an amount of 1.0 g, 1.0 g of sodium ascorbate, 0.2 g of sodium carboxymethyl cellulose, and 0.2 g of diglycerol monooleate (SUNSOFT Q-17D, having an HLB value of 7.5) were mixed, and water was added. The resulting mixture was kneaded. The kneaded material obtained was extrusion-granulated using a 1.2 mm Φ screen, and dried to obtain a particulate solid composition. The resulting particulate solid composition in an amount of 1 g was stored at 40°C and 75%RH for two months, and the storage stability of the reduced coenzyme Q10 was evaluated on the basis of the QH residual rate.

(Example 7)

A reduced coenzyme Q10 Form II crystal in an amount of 1.0 g, 1.0 g of sodium ascorbate, 0.2 g of potato starch, and 0.2 g of diglycerol monooleate (SUNSOFT Q-17D, having an HLB value of 7.5) were mixed, and water was added. The resulting mixture was kneaded. The kneaded material obtained was extrusion-granulated using a 1.2 mm Φ screen, and dried to obtain a particulate solid composition. The resulting particulate solid composition in an amount of 1 g was stored at 40°C and 75%RH for two months, and the storage stability of the reduced coenzyme Q10 was evaluated on the basis of the QH residual rate.

The QH residual rates after storage at 40°C for two months in Example 1 (in which the emulsifier A-1 was used), Example 5, Example 6, and Example 7 are shown in Table 6.

[Table 6]

|  | Binder | QH Residual Rate (%) |
|---|---|---|
| Example 1 (A-1) | hydroxypropyl cellulose | 95.4 |
| Example 5 | hydroxypropylmethyl cellulose | 96.6 |
| Example 6 | sodium carboxymethyl cellulose | 94.0 |
| Example 7 | potato starch | 96.3 |

The QH residual rate after storage at 40°C for three months in Example 1 (in which the emulsifier A-1 was used) is shown in Table 7.

[Table 7]

|  | Granulated or Non-granulated | QH Residual Rate (%) |
|---|---|---|
| Example 1 (A-1) | Granulated | 95.3 |

As shown in Table 7, the reduced coenzyme Q10 in the composition obtained by granulation had excellent storage stability.

(Example 8)

A reduced coenzyme Q10 Form II crystal in an amount of 150 g, 135 g of sodium erythorbate, and 15 g of diglycerol monooleate (SUNSOFT Q-17D, having an HLB value of 7.5) were mixed. The mixture obtained was introduced into a high-shear granulator (SPGJ-2TG, manufactured by Dalton Corporation) and mixed, and then, 20 mL of water was added. The resulting mixture was granulated, and dried to obtain a particulate solid composition. The resulting particulate solid composition in an amount of 1 g was stored at 40°C and 75%RH for two months, and the storage stability of the reduced coenzyme Q10 was evaluated on the basis of the QH residual rate. The measurement result of the QH residual rate is shown in Table 8.

[Table 8]

|  | QH Residual Rate (%) |
|---|---|
| Example 8 | 97.9 |

(Example 9)

Particulate solid compositions were obtained in the same manner as in Example 1 except that the unsaturated fatty acid-type emulsifiers shown in Table 9 were used. The resulting particulate solid composition in an amount of 1 g was stored under the conditions described in Table 10, and the storage stability of the reduced coenzyme Q10 was evaluated on the basis of the QH residual rate. The measurement result of the QH residual rate is shown in Table 10.

[Table 9]

|  | Emulsifier | Product Name |
|---|---|---|
| Example 9-1 | monoglycerol monooleate | SUNSOFT No. 8070V |
| Example 9-2 | diglycerol monooleate | SUNSOFT Q-17D |
| Example 9-3 | diglycerol monooleate | POEM DO-100V |
| Example 9-4 | tetraglycerol pentaoleate | SY-Glyster PO-3 S |
| Example 9-5 | pentaglycerol trioleate | SUNSOFT A-173E |
| Example 9-6 | hexaglycerol monooleate | SY-Glyster MO-5S |
| Example 9-7 | decaglycerol pentaoleate | SUNSOFT Q-175S |
| Example 9-8 | condensed pentaglycerol ricinoleate | SUNSOFT No. 818R |
| Example 9-9 | sucrose oleate | Ryoto Sugar Ester 0-170 |
| Example 9-10 | sorbitan monooleate | SUNSOFT No. 81S |
| Example 9-11 | propylene glycol monooleate | SUNSOFT No. 25-ODV |
| Example 9-12 | polyoxyethylene sorbitan monooleate | Tween 80 |
| Example 9-13 | sucrose erucate | Ryoto Sugar Ester ER-290 |
| Example 9-14 | enzymolytic soybean lecithin | SUNLECITHIN A |

[Table 10]

| | Storage Conditions | QH Residual Rate (%) |
|---|---|---|
| Example 9-1 | together with silica gel in a hermetically sealed aluminum laminate bag, at 25°C and 60% RH for 3 months | 98.6 |
| Example 9-2 | | 98.9 |
| Example 9-3 | | 98.7 |
| Example 9-4 | | 96.1 |
| Example 9-5 | | 98.3 |
| Example 9-6 | | 98.2 |
| Example 9-7 | | 98.6 |
| Example 9-8 | | 97.2 |
| Example 9-9 | | 95.6 |
| Example 9-10 | | 98.5 |
| Example 9-11 | | 95.4 |
| Example 9-12 | | 98.4 |
| Example 9-13 | together with silica gel in a hermetically sealed aluminum laminate bag, at 40°C and 75% RH for 1 month | 95.9 |
| Example 9-14 | in an opened system, at 25°C and 60% RH for 3 months | 98.1 |

(Example 10)

Particulate solid compositions were obtained in the same manner as in Example 1 except that the saturated fatty acid-type emulsifiers shown in Table 11 were used. The resulting particulate solid composition in an amount of 1 g was stored under the conditions described in Table 12, and the storage stability of the reduced coenzyme Q10 was evaluated on the basis of the QH residual rate. The measurement result of the QH residual rate is shown in Table 12.

[Table 11]

| | emulsifier | Product Name |
|---|---|---|
| Example 10-1 | monoglycerol monolaurate | SUNSOFT No. 750 |
| Example 10-2 | tetraglycerol pentastearate | SY-Glyster PS-3 S |
| Example 10-3 | decaglycerol monobehenate | Ryoto Polygly Ester B-70D |
| Example 10-4 | sorbitan monostearate | POEM S-60V |
| Example 10-5 | tetraglycerol pentastearate | SY-Glyster PS-3 S |
| Example 10-6 | sorbitan tristearate | POEM S-65V |

[Table 12]

| | Storage Conditions | QH Residual Rate (%) |
|---|---|---|
| Example 10-1 | together with silica gel in a hermetically sealed aluminum laminate bag, at 25°C and 60% RH for 3 months | 93.7 |
| Example 10-2 | | 93.4 |
| Example 10-3 | | 93.7 |
| Example 10-4 | | 92.6 |
| Example 10-5 | together with silica gel in a hermetically sealed aluminum laminate bag, at 40°C and 75% RH for 1 month | 93.5 |
| Example 10-6 | | 94.2 |

(Example 11)

Particulate solid compositions were obtained in the same manner as in Example 1 except that the emulsifiers shown in Table 13 were used. The resulting particulate solid composition in an amount of 1 g was stored under the conditions described in Table 14, and the storage stability of the reduced coenzyme Q10 was evaluated on the basis of the QH residual rate. The measurement result of the QH residual rate is shown in Table 14. In addition, Table 14 shows the properties of the emulsifiers.

[Table 13]

|  | Emulsifier | Product Name |
|---|---|---|
| Example 11-1 | monoglycerol monooleate | SUNSOFT No. 8070V |
| Example 11-2 | diglycerol monooleate | SUNSOFT Q-17D |
| Example 11-3 | diglycerol monooleate | POEM DO-100V |
| Example 11-4 | tetraglycerol pentaoleate | SY-Glyster PO-3 S |
| Example 11-5 | pentaglycerol trioleate | SUNSOFT A-173E |
| Example 11-6 | hexaglycerol monooleate | SY-Glyster MO-5S |
| Example 11-7 | decaglycerol pentaoleate | SUNSOFT Q-175S |
| Example 11-8 | condensed pentaglycerol ricinoleate | SUNSOFT No. 818R |
| Example 11-9 | sucrose oleate | Ryoto Sugar Ester 0-170 |
| Example 11-10 | sorbitan monooleate | SUNSOFT No. 81S |
| Example 11-11 | propylene glycol monooleate | SUNSOFT No. 25-ODV |
| Example 11-12 | polyoxyethylene sorbitan monooleate | Tween 80 |
| Example 11-13 | diglycerol monocaprylate | SUNSOFT Q-10D |
| Example 11-14 | hexaglycerol monocaprylate | SUNSOFT Q-81F |
| Example 11-15 | decaglycerol monolaurate | SUNSOFT M-12J |
| Example 11-16 | pentaglycerol monostearate | SUNSOFT A-181E |
| Example 11-17 | polyoxyethylene sorbitan monolaurate | Tween 20 |
| Example 11-18 | polyoxyethylene sorbitan monostearate | Tween 60 |
| Example 11-19 | monoglycerol monocaprylate | SUNSOFT No.700P-2 |
| Example 11-20 | diglycerol monolaurate | SUNSOFT Q-12D |
| Example 11-21 | diglycerol monomyristate | POEM DM-100 |

[Table 14]

|  | Properties | Storage Conditions | QH Residual Rate (%) |
|---|---|---|---|
| Example 11-1 | soft solid | together with silica gel | 98.6 |
| Example 11-2 | viscous liquid | | 98.9 |

(continued)

| | Properties | Storage Conditions | QH Residual Rate (%) |
|---|---|---|---|
| Example 11-3 | viscous liquid | in a hermetically sealed aluminum laminate bag, at 25°C and 60% RH for 3 months | 98.7 |
| Example 11-4 | liquid | | 96.1 |
| Example 11-5 | paste | | 98.3 |
| Example 11-6 | viscous liquid | | 98.2 |
| Example 11-7 | viscous liquid | | 98.6 |
| Example 11-8 | viscous liquid | | 97.2 |
| Example 11-9 | sticky liquid | | 95.6 |
| Example 11-10 | viscous liquid | | 98.5 |
| Example 11-11 | liquid | | 95.4 |
| Example 11-12 | viscous liquid | | 98.4 |
| Example 11-13 | viscous liquid | | 99.0 |
| Example 11-14 | viscous liquid | | 99.0 |
| Example 11-15 | viscous liquid | | 98.9 |
| Example 11-16 | pellets | | 95.7 |
| Example 11-17 | viscous liquid | together with silica gel in a hermetically sealed aluminum laminate bag, at 40°C and 75% RH for 1 month | 97.0 |
| Example 11-18 | viscous liquid | | 95.4 |
| Example 11-19 | liquid | | 99.1 |
| Example 11-20 | viscous liquid | | 99.4 |
| Example 11-21 | paste | | 99.3 |

(Example 12)

Particulate solid compositions were obtained in the same manner as in Example 1 except that the emulsifiers shown in Table 15 were used. The resulting particulate solid composition in an amount of 1 g, together with silica gel in a hermetically sealed aluminum laminate bag, was stored at 25°C and 60%RH for three months, and the storage stability of the reduced coenzyme Q10 was evaluated on the basis of the QH residual rate. The measurement result of the QH residual rate is shown in Table 16. In addition, Table 16 shows the properties of the emulsifiers.

[Table 15]

| | Emulsifier | Product Name |
|---|---|---|
| Example 12-1 | monoglycerol monolaurate | SUNSOFT No.750 |
| Example 12-2 | tetraglycerol pentastearate | SY-Glyster PS-3 S |
| Example 12-3 | decaglycerol monobehenate | Ryoto Polyglyester B-70D |
| Example 12-4 | mono- and di-glycerol monostearates | SUNSOFT No.118 |

[Table 16]

| | Properties | QH residual rate (%) |
|---|---|---|
| Example 12-1 | particulate powder | 93.7 |
| Example 12-2 | flakes | 93.4 |

(continued)

|  | Properties | QH residual rate (%) |
|---|---|---|
| Example 12-3 | flakes | 93.7 |
| Example 12-4 | particulate powder | 94.7 |

(Example 13)

A reduced coenzyme Q10 Form II crystal in an amount of 49.5 g, 36.0 g of sodium erythorbate, and 4.5 g of diglycerol monooleate (SUNSOFT Q-17D, manufactured by Taiyo Kagaku Co., Ltd.,) were used as raw materials, granulated by fluidized-bed granulation using pulvis GB22 (manufactured by Yamato Scientific Co., Ltd.), and dried to obtain a particulate solid composition. The resulting particulate solid composition in an amount of 1 g, together with silica gel in a hermetically sealed aluminum laminate bag, was stored at 25°C and 60%RH for three months, and the storage stability of the reduced coenzyme Q10 was evaluated on the basis of the QH residual rate. The QH residual rate was 98.5%.

(Example 14)

A reduced coenzyme Q10 Form II crystal in an amount of 3.0 g, 2.2 g of sodium erythorbate, 0.3 g of soybean lecithin (SLP-paste, manufactured by Tsuji Oil Mills Co., Ltd.), and 0.5 g of yeast cell wall (yeast wrap, manufactured by Mitsubishi Corporation Life Sciences Limited) were used as raw materials, granulated by high-shear granulation using a coffee mill, and dried to obtain a particulate solid composition. The resulting particulate solid composition in an amount of 1 g was stored at 25°C and 60%RH for three months, and the storage stability of the reduced coenzyme Q10 was evaluated on the basis of the QH residual rate. The QH residual rate was 97.9%.

(Example 15)

To evaluate the formulability of the particulate solid composition and the storage stability in the formulated product, the following experiment was performed.

The particulate solid composition obtained in the same manner as in Example 8, in an amount of 10 g, 14.85 g of microcrystalline cellulose, and 0.15 g of magnesium stearate were mixed. The powder mixture in an amount of 250 mg was introduced into a die and punch tableting tool for a Φ8 round tablet, and compressed into a tablet under a force of 6.5 kN. The tablet produced was stored at 25°C and 60%RH for three months together with silica gel in a hermetically sealed aluminum laminate bag, and the storage stability of the reduced coenzyme Q10 was evaluated on the basis of the QH residual rate. The QH residual rate was 99.1%.

(Reference Example)

The reduced coenzyme Q10 Form I crystal (Form I - crystal), the particulate solid composition produced using the reduced coenzyme Q10 Form I crystal in the same manner as in Comparative Example 1 (Form I - stabilized composition), the reduced coenzyme Q10 Form II crystal (Form II - crystal), and the particulate solid composition produced using the reduced coenzyme Q10 Form II crystal in the same manner as in Example 1 (A-1) (Form II - stabilized composition) were stored at 25°C and 60%RH for 14 days. From the QH residual rate after storage, the half-life period, which is the number of days required for the QH residual rate to reach 50%, and the stabilization degree, which is the ratio of the half-life period of the stabilized composition to the half-life period of each crystal, were determined and shown in Table 17.

[Table 17]

|  | Half-life Period (days) | Stabilization degree (with respect to each crystal) |
|---|---|---|
| Form I - crystal | 14 | - |
| Form I - stabilized composition | 23 | 1.6 fold |
| Form II - crystal | 189 | - |
| Form II - stabilized composition | 3230 | 17 fold |

As shown in Table 17, incorporation of the reduced coenzyme Q10 Form I crystal into the stabilized composition resulted in only 1.6-fold higher stability. On the other hand, incorporation of the reduced coenzyme Q10 Form II crystal into the stabilized composition resulted in 17-fold higher stability, which was an unexpected result. These results indicate that the stabilization of the reduced coenzyme Q10 in the Examples of the present invention was due to a hitherto unrevealed property unique to the reduced coenzyme Q10 Form II crystal. Due to the synergistic effect of the high stability of the reduced coenzyme Q10 Form II crystal (14 times more stable than the Form I crystal) and the high stabilizing effect of the incorporation into the stabilized composition (17 times more stable), which was observed only with the Form II crystal, the Form II - stabilized composition was 140 times more stable than the Form I - stabilized composition, resulting in a high stability commensurate with production costs.

All publications, patents, and patent applications cited herein are incorporated herein by reference in their entirety.

The upper and/or lower limits of the numerical ranges herein may be arbitrarily combined to define a preferred range. For example, any upper limit and any lower limit of the numerical ranges may be combined to define a preferred range. Any upper limits of the numerical ranges may be combined to define a preferred range. Any lower limits of the numerical ranges may be combined to define a preferred range. A numerical range described herein with the term "to" includes the values presented before and after the term "to" as a lower limit and an upper limit, respectively.

It should be understood that throughout the present description, expressions in the singular include the plural, unless otherwise specified. Accordingly, the singular articles (e.g., "a", "an", and "the" in English) include plural references unless otherwise specified.

Although the present embodiments are described in detail above, the specific configuration is not limited to these embodiments, and the present disclosure encompasses any design modification within the scope which does not depart from the spirit of the present disclosure.

**Claims**

1.   A solid composition comprising a reduced coenzyme Q10 Form II crystal, an emulsifier, and an antioxidant.

2.   The solid composition according to claim 1, wherein the emulsifier is one or more selected from the group consisting of: ester compounds of polyols and fatty acids optionally having substituents, wherein the polyols are selected from the group consisting of monoglycerol, polyglycerol, sorbitan, polyoxyethylene sorbitan, sucrose, propylene glycol, polypropylene glycol, ethylene glycol, and polyethylene glycol; and lecithin.

3.   The solid composition according to claim 1 or 2, comprising 1 part by weight or more and 9900 parts by weight or less of the emulsifier with respect to 100 parts by weight of the crystal.

4.   The solid composition according to any one of claims 1 to 3, wherein the amount of the emulsifier contained in the solid composition is 1% by weight or more and 99% by weight or less.

5.   The solid composition according to any one of claims 1 to 4, wherein the antioxidant is one or more selected from the group consisting of ascorbic acid, ascorbic acid salts, erythorbic acid, and erythorbic acid salts.

6.   The solid composition according to any one of claims 1 to 5, further comprising a binder.

7.   The solid composition according to claim 6, wherein the binder is one or more selected from the group consisting of celluloses and starches.

8.   The solid composition according to claim 7, wherein the binder is one or more selected from the group consisting of hydroxypropyl cellulose and hydroxypropylmethyl cellulose.

9.   The solid composition according to any one of claims 6 to 8, wherein the solid composition is a particulate solid composition.

10.  The solid composition according to any one of claims 1 to 9, wherein the solid composition is free of a carbonate containing a sodium cation and/or a calcium cation.

11.  The solid composition according to any one of claims 1 to 10, wherein the solid composition is free of porous calcium silicate.

12. A method for producing a particulate solid composition comprising a reduced coenzyme Q10 Form II crystal, an emulsifier, an antioxidant, and a binder, the method comprising
granulating a mixture comprising the reduced coenzyme Q10 Form II crystal, the emulsifier, the antioxidant, the binder, and a liquid binder.

13. The method according to claim 12, wherein the granulating is performed by an extrusion granulation method, a high-shear granulation method, or a fluidized-bed granulation method.

## INTERNATIONAL SEARCH REPORT

| International application No. |
| --- |
| **PCT/JP2023/010017** |

| A. CLASSIFICATION OF SUBJECT MATTER |
| --- |
| *A61K 31/09*(2006.01)i; *A23L 5/00*(2016.01)i; *A23L 29/10*(2016.01)i; *A23L 33/10*(2016.01)i; *A61K 9/14*(2006.01)i; *A61K 47/14*(2017.01)i; *A61K 47/22*(2006.01)i; *A61K 47/24*(2006.01)i; *A61K 47/36*(2006.01)i; *A61K 47/38*(2006.01)i; *A61P 3/00*(2006.01)i; *A61P 9/00*(2006.01)i; *A61P 39/06*(2006.01)i; *A61P 43/00*(2006.01)i<br>FI: A61K31/09; A23L5/00 A; A23L5/00 D; A23L29/10; A23L33/10; A61K9/14; A61K47/14; A61K47/22; A61K47/24; A61K47/36; A61K47/38; A61P3/00; A61P9/00; A61P39/06; A61P43/00 105 |
| According to International Patent Classification (IPC) or to both national classification and IPC |

| B. FIELDS SEARCHED |
| --- |
| Minimum documentation searched (classification system followed by classification symbols) |
| A61K31/09; A23L5/00; A23L29/10; A23L33/10; A61K9/14; A61K47/14; A61K47/22; A61K47/24; A61K47/36; A61K47/38; A61P3/00; A61P9/00; A61P39/06; A61P43/00 |
| Documentation searched other than minimum documentation to the extent that such documents are included in the fields searched |
| Published examined utility model applications of Japan 1922-1996<br>Published unexamined utility model applications of Japan 1971-2023<br>Registered utility model specifications of Japan 1996-2023<br>Published registered utility model applications of Japan 1994-2023 |
| Electronic data base consulted during the international search (name of data base and, where practicable, search terms used) |
| CAplus/REGISTRY/MEDLINE/EMBASE/BIOSIS (STN), JSTPlus/JMEDPlus/JST7580 (JDreamIII) |

| C. DOCUMENTS CONSIDERED TO BE RELEVANT |
| --- |

| Category* | Citation of document, with indication, where appropriate, of the relevant passages | Relevant to claim No. |
| --- | --- | --- |
| Y | JP 2007-145831 A (KANEKA CORP.) 14 June 2007 (2007-06-14)<br>claims, paragraphs [0032], [0047], [0051], example 2, production example 5 | 1-13 |
| Y | WO 2008/129980 A1 (KANEKA CORP.) 30 October 2008 (2008-10-30)<br>claims, paragraphs [0015], [0152], examples 2-8 | 1-13 |
| Y | WO 2012/176842 A1 (KANEKA CORP.) 27 December 2012 (2012-12-27)<br>claims, paragraphs [0030], [0053]-[0055] | 1-13 |
| Y | WO 2015/122531 A1 (KANEKA CORP.) 20 August 2015 (2015-08-20)<br>claims, paragraphs [0016], [0020]-[0027], [0032] | 1-13 |
| A | WO 2006/075502 A1 (KANEKA CORP.) 20 July 2006 (2006-07-20)<br>entire text | 1-13 |

☑ Further documents are listed in the continuation of Box C.     ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **15 May 2023** | **30 May 2023** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**

| | International application No. |
|---|---|
| | **PCT/JP2023/010017** |

**C. DOCUMENTS CONSIDERED TO BE RELEVANT**

| Category* | Citation of document, with indication, where appropriate, of the relevant passages | Relevant to claim No. |
|---|---|---|
| A | WO 2005/033054 A1 (KANEKA CORP.) 14 April 2005 (2005-04-14)<br>entire text | 1-13 |

Form PCT/ISA/210 (second sheet) (January 2015)

**INTERNATIONAL SEARCH REPORT**
Information on patent family members

International application No.

**PCT/JP2023/010017**

| Patent document cited in search report | | | Publication date (day/month/year) | Patent family member(s) | | | Publication date (day/month/year) |
|---|---|---|---|---|---|---|---|
| JP | 2007-145831 | A | 14 June 2007 | US | 2007/0104701 | A1 | |
| | | | | claims, paragraphs [0042], [0058], [0062], example 2, Dosage form example 5 | | | |
| WO | 2008/129980 | A1 | 30 October 2008 | US | 2010/0092560 | A1 | |
| | | | | claims, paragraphs [0016], [0168], examples 2-8 | | | |
| WO | 2012/176842 | A1 | 27 December 2012 | EP | 2725004 | A1 | |
| | | | | claims, paragraphs [0030], [0054]-[0056] | | | |
| | | | | US | 2014/0120073 | A1 | |
| | | | | US | 2016/0289152 | A1 | |
| | | | | AU | 2012274434 | A | |
| | | | | CA | 2837695 | A1 | |
| | | | | CN | 103635452 | A | |
| | | | | KR | 10-2014-0061350 | A | |
| | | | | BR | 112013032641 | A | |
| | | | | TW | 201311635 | A | |
| WO | 2015/122531 | A1 | 20 August 2015 | TW | 201613561 | A | |
| WO | 2006/075502 | A1 | 20 July 2006 | EP | 1829538 | A1 | |
| | | | | US | 2006/0147542 | A1 | |
| | | | | CA | 2588925 | A1 | |
| | | | | CN | 101087598 | A | |
| | | | | AU | 2005324603 | A | |
| | | | | KR | 10-2007-0091680 | A | |
| | | | | RU | 2007128342 | A | |
| | | | | TW | 200635605 | A | |
| WO | 2005/033054 | A1 | 14 April 2005 | EP | 1666445 | A1 | |
| | | | | US | 2005/0074860 | A1 | |
| | | | | AU | 2004278194 | A | |
| | | | | KR | 10-2006-0130016 | A | |
| | | | | CN | 1849287 | A | |
| | | | | TW | 200510289 | A | |

Form PCT/ISA/210 (patent family annex) (January 2015)

**REFERENCES CITED IN THE DESCRIPTION**

**Patent documents cited in the description**

- WO 2006075502 A
- WO 2008129980 A
- WO 2012176842 A
- WO 2015122531 A
- JP 2010189337 A
- JP 60221078 A
- JP 2022042302 A